# EUROPÄISCHE PATENTSCHRIFT

(11) **EP 0 513 672 B1**
(45) Veröffentlichungstag und Bekanntmachung des Hinweises auf die Patenterteilung: **02.08.1995**
(21) Anmeldenummer: 92107748.3
(22) Anmeldetag: 08.05.1992
(51) Int. Cl.: A61M 1/34

(54) **Vorrichtung zur Behandlung von Blut mit volumetrischer Flüssigkeitsbilanzierung**
Blood treatment device with volume balanced flow
Dispositif de traitement du sang avec équilibrage volumique du débit

(30) Priorität: 17.05.1991 DE 4116178
(43) Veröffentlichungstag der Anmeldung: 19.11.1992
(73) Patentinhaber: Fresenius AG, 61350 Bad Homburg (DE)
(72) Erfinder: Beden, Josef, W-6503 Mainz-Kastel (DE); Flaig, Hans-Jürgen, Dr., W-6420 Lauterbach (DE); Polaschegg, Hans-Dietrich, Dr., W-6370 Oberursel 4 (DE); Steinbach, Bernd, Dr., W-6380 Bad Homburg (DE)
(74) Vertreter: Fuchs Mehler Weiss

(56) Entgegenhaltungen:
- EP-A- 0 303 765
- WO-A-88/02641
- DE-A- 2 634 238
- FR-A- 2 457 694

## Beschreibung

Die Erfindung betrifft eine Vorrichtung zur Behandlung von Blut in einem extrakorporalen Blutkreislauf, der durch die Blutkammer eines Filters gelegt ist, der durch eine semipermiable Membran in die Blutkammer und eine Filtratkammer geteilt ist, mit einer Filtratleitung, die von der Filtratkammer abgeht und in die eine Filtratpumpe eingeschaltet ist, mit einer Substituatquelle, von der eine Substituatleitung abgeht, in die eine Substituatpumpe eingeschaltet ist, mit einer Bilanzierkammer, die durch eine flexible Wand in eine erste und eine zweite Bilanzierkammerhälfte geteilt ist, wobei die erste Hälfte mit der Filtratleitung und die zweite Hälfte mit der Substituatleitung verbunden sind, mit einer Filtratauslaßleitung, die von der ersten Bilanzkammerhälfte zu einem Auslaß abgeht, und einer Substituatauslaßleitung, die von der zweiten Bilanzkammerhälfte abgeht und mit dem extrakorporalen Blutkreislauf zu verbinden ist, einer ersten Absperrklemme, die in der Filtratleitung angeordnet ist, einer zweiten Filtratklemme, die an der Filtratauslaßleitung angeordnet ist, einer dritten Absperrklemme, die an der Substituatleitung angeordnet ist, und einer vierten Absperrklemme, die an der Substituatauslaßleitung angeordnet ist, und einer Steuereinheit, die die Pumpen und die Absperrklemmen in vorbestimmter Weise aktiviert.

Eine derartige Vorrichtung ist beispielsweise aus der DE-A-2 634 238 bekannt.

Bei der Blutbehandlung, insbesondere der Behandlung von Urämie mit Geräten zur Hämodialyse, Hämofiltration, Hämodiafiltration oder Peritonealdialyse, bei der kontinuierlichen arterio-venösen Hämofiltration und bei der kontinuierlichen veno-venösen Hämofiltration sowie bei der Plasmafiltration besteht die Notwendigkeit einer exakten Bilanzierung von entzogener Flüssigkeit einerseits zur zugeführter Flüssigkeit andererseits und der zu ultrafiltrierenden Mengen über die gesamte Behandlungszeit.

Bei der Hämodialyse erfolgt die Bilanzierung heute üblicherweise über Bilanzkammern, wie sie beispielsweise in den deutschen Offenlegungsschriften 25 44 258, 26 34 238, 28 38 414 oder der US-Patentschrift 40 54 522 beschrieben sind. Desgleichen sind Hämodialysemaschinen am Markt erhältlich, beispielsweise diejenige der Anmelderin mit der Bezeichnung A 2008, die ebenfalls bilanzierend Dialysierflüssigkeit einem Dialysator zuführen, wobei kontrolliert ultrafiltriert wird.

Sämtliche bilanzierende Hämodialysevorrichtungen weisen zwei Bilanzkammern auf, die wechselweise mit einer Dialysierflüssigkeitsquelle einerseits und einem Auslaß andererseits zur Füllung bzw. Entleerung mit Dialysierflüssigkeit und andererseits mit dem Dialysefilter verbunden sind, um frische Dialysierflüssigkeit von einer Bilanzkammerhälfte durch den Dialysator hindurch in die andere Bilanzkammerhälfte zu pumpen, wobei die Bilanzkammer durch eine flexible Wand in diese beiden Hälften geteilt ist. Demzufolge wird in letztem Fall ein geschlossener, volumenkonstanter Kreislauf aufgebaut, aus dem kontrolliert Ultrafiltrat entnommen wird. Erst durch den Einsatz derart bilanzierender Einrichtungen war es möglich, den üblichen Pumpenfehler von 10 % auf unter 1 % zu drücken, sodaß hierdurch hochleistungsfähige Membranen wirksam eingesetzt werden konnten.

Während - wie vorstehend beschrieben - der innere Kreislauf der Bilanzierungsanordnung geschlossen ist, ist bei den bekannten Einrichtungen der Zuführungskreis gegenüber der Atmosphäre geöffnet, was dazu führt, daß beide Kreise sich in der Praxis nicht exakt abstimmen lassen. Demzufolge arbeitet eine Reihe von bekannten Vorrichtungen mit einem Überschuß an Dialysierflüssigkeit im Zuführungskreis, der permanent in den Abfluß gefördert wird. Dieses ist natürlich aus Gründen der Kostenersparnis unerwünscht.

Gegenüber der Hämodialyse, bei der eine Behandlung des Bluts mit Hilfe der Dialysierflüssigkeit erfolgt, werden bei der Hämofiltration dem Blutkreislauf Filtrat entzogen und eine Substituatlösung zugeführt. Dabei wird die zugeführte Substituatlösung gegenüber dem abgezogenen Filtrat bilanziert, was üblicherweise auf einer Waage erfolgt. Eine derartige Lösung ist beispielsweise in der Deutschen Patentschrift 37 43 272 beschrieben. Derartige Waagen sind zum einen relativ teuer und zum anderen relativ empfindlich und müssen daher exakt überwacht werden. Letzteres ist in der genannten Patentschrift beschrieben. Auf der anderen Seite hat die Hämofiltration und die Plasmafiltration den Vorteil, daß das gesamte Reinigungsverfahren in sich geschlossen gegenüber der Atmosphäre durchgeführt werden kann, d.h. keine Keime in die Blutbahn gelangen können.

Eine mittels einer Waage zur Durchführung einer Plasmapherese bilanzierte Vorrichtung zeigt im übrigen die bekannt gewordene DE-Z "Biomed-Technik", Band 29, Heft 6/1984, S. 146-150.

Eine Vorrichtung zur Ultrafiltration bei der Hämodialyse ist bekannt aus der DE 38 37 498 C2. Darin wird beschrieben, daß der in der Vorrichtung vorgesehene Luftabscheider solange mit Dialysierflüssigkiet gefüllt wird, bis eine Sensormarke erreicht ist. Mit der Vorrichtung soll eine möglichst kontinuierliche Ultrafiltration möglich werden. Ein Problem wird darin gesehen, daß die Dialysierflüssigkeit mit Luft in Berührung kommt und damit unter Umständen mit darin enthaltenen Keimen oder dergleichen.

Der Erfindung liegt vor dem aufgezeigten Hintergrund die Aufgabe zugrunde, eine Vorrichtung zur Blutreinigung zur Verfügung zu stellen, die geschlossen gegenüber dem Blutkreislauf auf einfache Weise Filtrat und Substituat gegeneinander bilanziert und darüber hinaus eine Ultrafiltration bzw. Überwässerung ermöglicht.

Die Lösung der Aufgabe erfolgt dadurch, daß in der Filtratleitung eine erste Ausgleichskammer und in der Substituatleitung eine zweite Ausgleichskammer stromauf der ersten bzw. dritten Klemme vorgesehen sind, wobei das Aufnahmevolumen der Ausgleichskammern jeweils größer ist als das Innenvolumen der Bilanzkammer und daß die Ausgleichskammern jeweils von einer stetig Druck auf sie ausübenden Kompressionseinrichtung beaufschlagt sind.

Die erfindungsgemäße Vorrichtung weist zunächst den Vorteil auf, daß sie auf einfache Weise im geschlossenen System zu bilanzieren vermag. Hierzu ist eine Bilanzkammer vorgesehen, in deren beiden Hälften Filtrat gegen Substituat bilanziert wird.

Diese Bilanzkammer weist einen Innenraum mit einem Innenvolumen auf, der durch eine flexible Wand in zwei Bilanzkammerteile oder -hälften geteilt ist, wobei wechselweise eine der beiden Flüssigkeiten den gesamten Innenraum unter Verdrängung der anderen Flüssigkeit einnehmen kann.

Diese Flüssigkeiten, nämlich Filtat und Substituat, werden wechselweise jeweils aus einer Ausgleichskammer mit Hilfe einer Druckeinrichtung zugeführt. Diese Ausgleichskammer weist ein größeres Volumen als der Innenraum der Bilanzkammer auf, wobei das Verhältnis dieser beiden Volumina vorteilhafterweise in einem Bereich von 1:1,2 - 1:1,5 liegt. Diese Ausgleichskammern, die üblicherweise in einem flexiblen Beutel vorgesehen sind, werden mittels der Filtratpumpe bzw. Substituatpumpe im wesentlichen gefüllt und nach Öffnen der jeweiligen Absperrklemmen mit Hilfe der Druckeinrichtung in die eine Bilanzkammerhälfte entleert, wobei sich gleichzeitig die andere Bilanzkammer unter der Druckeinwirkung ebenfalls in die jeweilige Ablaufleitung entleert. Die Absperrklemmen sind dabei so geschaltet, daß bei zulaufendem Filtrat die Substituatlösung abläuft, während bei zulaufendem Substituat die Filtratlösung abläuft. Demzufolge werden wechselweise zwei Klemmenpaare in die jeweils andere Betriebslage umgeschaltet.

Die Regelung der Pumpendrehzahl erfolgt jeweils so, daß unabhängig vom mittleren Filtrat- bzw. Substituatfluß vor Öffnung der entsprechenden Absperrklemmen eine festgelegte, konstante Füllmenge in den Ausgleichskammern vorliegt. Durch vorteilhafte Wahl dieses Flüssigkeitsvolumens kann eine möglichst gleichmäßige Förderrate der Pumpen über den Gesamtzyklus erreicht werden, und dennoch der fehlerfreie Ablauf einfach überwacht werden.

Die vorstehende Verfahrensweise betrifft eine 1:1-Bilanzierung, d.h. es werden äquivalente Mengen an Filtrat und Substituat während der Behandlung zu-und abgefördert.

Wenn andererseits eine Ultrafiltration durchgeführt werden soll, also dem Patienten eine bestimmte Menge Flüssigkeit entnommen werden soll, werden erfindungsgemäß bestimmte Takte auf der Substituatseite ausgelassen. Dabei werden bei dem jeweils ausgewählten Takt die Substituatmengen nicht zum Patienten, sondern zur Substituatquelle zurückgefördert. In diesem Takt bleibt also die Auslaufklemme geschlossen und die Einlaufklemme geöffnet und es wird die in der Substituatkammer befindliche Substituatmenge durch Umkehrung des Laufs der Substituatpumpe zur Substituatquelle zurückgefördert. Die Anzahl der ausgewählten Takte multipliziert mit dem Volumen des Innenraums der Bilanzkammer gibt dabei die während der Behandlung dem Patienten entnommene Menge. Eine solche Menge kann im übrigen vor der Behandlung vorgewählt werden, was zur Folge hat, daß entsprechend einem vorgegebenen Programm bestimmte Fördertakte ausgelassen werden.

Die vorstehende Ultrafiltrationsbehandlungsweise gilt bei Patienten, die beispielsweise wegen Urämie überwässert sind.

Andererseits muß zur Regulierung des Säure/Basen-und Elektrolythaushalts bei einigen Patienten mehr entsprechendes Substituat zugeführt werden, als Filtrat abgeführt wird.

In einem solchen Fall kann die Filtratpumpe nicht in den Filter zurückfördern. Demzufolge dient das Ausgleichsreservoir bei dieser Ausführungsform als Auffangbehälter. Der Arbeitstakt sieht bei dieser Ausführungsform daher folgendermaßen aus: Zunächst pumpt die Filtratpumpe bzw. deren Ausgleichsgefäß unter Druckeinwirkung die Bilanzkammer mit Filtrat voll. Dabei sind das Filtrateinlaß- und das Substituatauslaßventil geöffnet, während die beiden anderen Ventile geschlossen sind. Beim Umschalten in die nächste Betriebsphase bleibt die Ventilkonfiguration auf der Filtratseite unverändert, während die beiden Ventile auf der Substituatseite in die andere Betriebsart umgeschaltet werden. Zugleich wird mit dem Umschaltvorgang die Filtratpumpe stillgesetzt. Die Substituatpumpe pumpt Substituat in die Bilanzkammer, was zu einem Verdrängen der Filtratmenge aus der Bilanzkammer in die Filtratausgleichskammer führt. Bei nächsten Umschalten wird wiederum die gleiche Menge Filtrat in die Bilanzkammer eingeführt, so daß bei diesem Takt bzw. Hub ein Teil Substituatlösung mehr bilanziert wurde.

Die Ausgleichskammern sind in einem Kunststoffbeutel vorgesehen, der in die jeweiligen Filtrat- bzw. Substituatleitungen eingeschaltet ist. Sie bestehen vorteilhafterweise aus dem gleichen Material wie die Leitungen (PVC und dergl.) und sind üblicherweise aus einem biegeschlaffen, jedoch wenig erweiterbaren Material gebildet. Ein solcher Beutel befindet sich zwischen einer Druck- oder Preßvorrichtung, die auf die Beutelwände einen vorbestimmten Druck ausübt.

Diese Preßvorrichtung besteht üblicherweise aus zwei Platten, die durch eine Feder oder dergl. zusammengedrückt werden. Üblicherweise wird auf der Filtratseite eine vorgespannte Feder mit einer Anfangskraft von ca. 100 N und einer Endkraft von 130 N eingesetzt, während auf der Substituatseite eine vorgespannte Feder mit einer Anfangskraft von 80 N und einer Endkraft von 130 N zum Einsatz kommt. Dabei beträgt die Steigung 1,5 bzw. 5 N/mm.

Die Federkraft reicht aus, um die teilweise gefüllte Ausgleichskammer innerhalb weniger Sekunden vollständig in die Bilanzkammer unter Verdrängen der jeweils anderen Lösung aus der Bilanzkammer zu drücken. Dies gilt insbesondere bei einem hohen venösen Rücklaufdruck auf der Substituatseite. Die vollständige Füllung der Bilanzkammer erfolgt durch die entsprechende Pumpe. Bei einem Bilanzkammervolumen von 25 ml und einem Volumen in der Ausgleichskammer von ca. 32-35 ml dauert das Pumpen mit den vorstehend genannten Druckvorrichtungen mindestens 2 sec.

Die Zeitdauer zur Befüllung der Bilanzierkammer sowie die Zeitdauer zur Befüllung der Ausgleichskammer hängt im wesentlichen ab von der Blutflußgeschwindigkeit in ml/min., da ca. 20-30% der Blutmenge durch Austausch von Filtrat/Substituat ersetzt wird.

Um Bilanzfehler zu vermeiden, werden beim Umschalten vorteilhafterweise sämtliche Ventile kurzzeitig in den inaktiven Zustand geschaltet, um dann in die jeweils andere Betriebsart überzugehen.

In einer bevorzugten Ausführungsform weist die Druckeinrichtung einen Längensensor auf, der den zu- und abnehmenden Durchmesser der Ausgleichskammer bestimmen kann. Der Beutel erweitert sich nämlich beim Füllen mit Flüssigkeit im wesentlichen in radialer Richtung und bläht sich zu einem zylinderähnlichen Gebilde auf. Dabei ist die Zunahme eines Durchmessers mit der zugeführten Flüssigkeitsmenge korreliert, d.h. aus der Veränderung des Längensensors kann die zugeführte Flüssigkeitsmenge und die Zuführungsrate bestimmt werden. Dieses Signal der Längenmessung wird nunmehr als Ist-Wert herangezogen und mit dem ursprünglich eingegebenen Soll-Wert, mit dem die jeweilige Pumpe fördert, verglichen, wobei geringe Abweichungen vom Soll-Ergebnis zu einer Regulierung der Pumpe führen können. In jedem Fall ist sicherzustellen, daß die Pumpen permanent laufen und soviel Flüssigkeit in den Ausgleichskammern vorliegt, daß die Bilanzkammer zu Beginn eines jeden Takts gefüllt werden kann. Beim Auftreten von Fehlern kann das Längensensorsignal zur Erkennung herangezogen werden.

Weitere Vorteile, Merkmale und Ausführungsformen der Erfindung sind in der nachfolgenden Zeichnung gezeigt und der Beschreibung erläutert.

Es zeigen:
- Figur 1: eine schematische Skizze einer Plasmafiltrationsvorrichtung mit der Filtrat/Substituat-Bilanzierungseinrichtung.
- Figur 2: ein Disposable in skizzierter Darstellung, das ein Schlauchsystem mit den beiden Ausgleichskammern und der Bilanzierkammer zeigt.

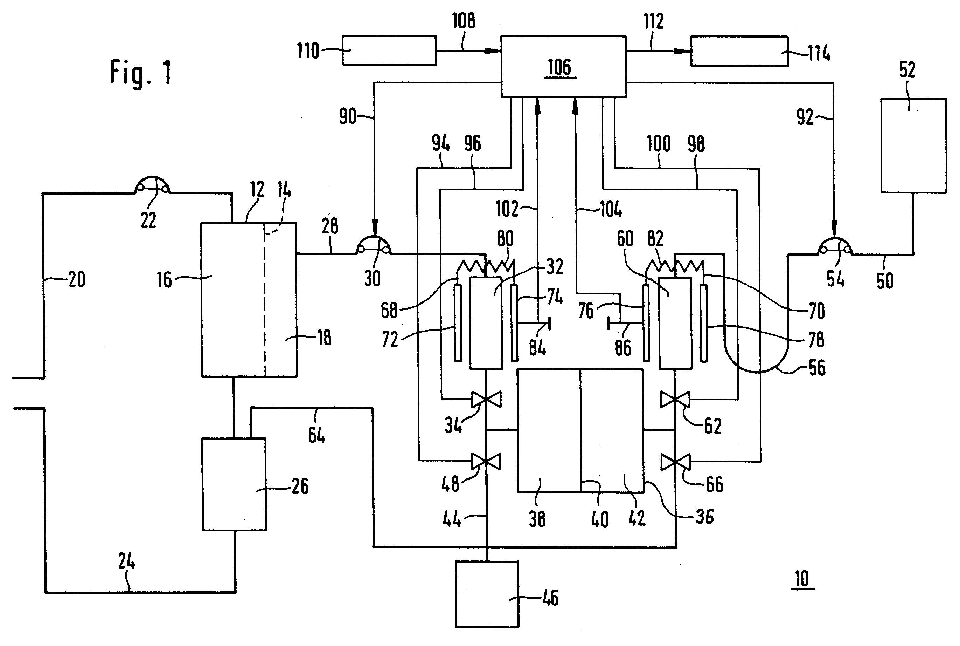

In Figur 1 ist eine Plasmafiltrationsanordnung 10 schematisch dargestellt. Diese Plasmafiltrationsanordnung 10 weist einen Plasmafilter 12 auf, der durch eine semipermiable Membran 14 in eine Blutkammer 16 und eine Filtratkammer 18 geteilt ist. Die Blutkammer 16 ist an ihren Einlauf mit einer Blutzuführungsleitung 20 vrbunden, in die eine Blutpumpe 22 eingeschaltet ist. Stromab der Blutkammer 16 ist eine Blutabflußleitung 24 zum Patienten zurückgeführt. In diese Blutabflußleitung ist eine Tropfkammer 26 eingeschaltet.

Auf der Filtratseite ist die Filtratkammer 18 mit einer ersten Filtratleitung 28 verbunden, in die eine Filtratpumpe 30 eingeschaltet ist. In diese Filtratleitung 28 ist stromab der Filtratpumpe 30 eine flexible Ausgleichskammer 32 in Form eines Kunststoffbeutels mit einem Innenvolumen von etwa 35 ml eingeschaltet. Stromab an diese Ausgleichskammer 32 schließt sich eine erste Absperrklemme 34 an, mit der die Filtratleitung geöffnet oder geschlossen werden kann.

Das Ende der Filtratleitung ist mit einer Bilanzkammer 36, und zwar mit der ersten Bilanzkammerhälfte 38 verbunden. Diese Bilanzkammerhälfte 38 wird durch eine in der Bilanzkammer 36 vorgesehene flexible Wand 40 gebildet, wobei zugleich auf der gegenüberliegenden Seite eine zweite Bilanzkammerhälfte 42 gebildet wird.

Die jeweiligen Bilanzkammerhälften können sich vollständig mit Flüssigkeit unter Verlagerung der flexiblen Wand 40 an die jeweils gegenüberliegende Bilanzkammerwand füllen, wobei die entsprechend andere Bilanzkammerhälfte vollständig entleert und damit aufgehoben wird. Demzufolge bilden die beiden Bilanzkammerhälften 38 und 42 das Innenvolumen der Bilanzkammer 36, wobei das Volumen der flexiblen Wand 40 zu berücksichtigen ist.

Von der ersten Bilanzkammerhälfte 38 geht eine Filtratablaßleitung 44 zum Ausguß ab, der im vorliegenden Fall als Filtratbeutel 46 ausgebildet ist. In die Filtratablaßleitung 44 ist weiterhin eine zweite Absperrklemme 48 eingeschaltet, mit der die Filtratablaßleitung 44 geschlossen oder geöffnet werden kann.

Die zweite Bilanzkammerhälfte 42 ist über eine Substituatleitung 50 mit einer Substituatquelle 52 verbunden. In diese Substituatquelle 52 ist eine Substituatpumpe 54 eingeschaltet, an die sich ein Heizteil 56 anschließt, mit der die Substituatflüssigkeit auf eine vorbestimmte Temperatur, üblicherweise die Körpertemperatur des Patienten, erwärmt wird. Stromab des Heizteils 56 ist eine zweite Ausgleichskammer 60 angeordnet, die entsprechend der ersten Ausgleichskammer 32 ausgebildet ist.

Hieran schließt sich eine dritte Absperrklemme 62 an, mit der die Substituatleitung 50 geöffnet oder geschlossen wird.

Vom Auslaß der als Substituatkammer ausgebildeten zweiten Bilanzkammerhälfte 42 geht eine Substituatauslaßleitung ab, die mit dem Blutkreislauf, im Beispielsfall mit der Tropfkammer 26 verbunden ist. Somit handelt es sich im vorliegenden Fall um eine Postdilutionsanordnung, wobei eine Prädilutionsanordnung bei der die Substituatauslaßleitung 64 mit der Blutzuführungsleitung 20 verbunden wäre, ebenfalls möglich ist.

In die Substituatauslaßleitung 64 ist eine vierte Absperrklemme 66 eingeschaltet, die in entsprechender Weise diese Leitung 64 öffnet bzw. schließt.

Die Ausgleichskammern 32 bzw. 60 weisen jeweils an ihrem Außenumfang eine Kompressionseinrichtung 68 und 70 auf, die im Beispielsfall aus jeweils zwei Platten 72 und 74 bzw. 76 und 78 bestehen, die jeweils über eine Feder 80 bzw. 82 komprimiert werden. Im Beispielsfall hat die Feder 80 eine Anfangscharakteristik von 100 N und eine Endcharakteristik von 130 N mit eine Steigung von 1,5 N/mm, während die zweite Feder 82 eine entsprechende Charakteristik von 80 N bzw. 130 N mit einer Steigung von 5 N/mm aufweist.

Mit dieser Kompressionsanordnung 68 und 70 werden die beiden Plattenpaare 74-78 gegen die Ausgleichskammern 32 und 60 mit der angegebenen Kraft gepreßt und entleeren diese im Entleerungsschritt.

Im Beispielsfall ist jeweils eine der Platten, nämlich die Platten 72 bzw. 78 am Rahmen der Anordnung fest angebracht, so daß nur die beiden anderen Platten 74 und 76 gegen die Federkraft bewegt werden können. Das Maß der Längsversetzung dieser beiden Platten 74, 76 kann jeweils mit einem Längensensor 84 und 86 ermittelt werden.

Die Filtratpumpe 30, die Substituatpumpe 54, die erste, zweite, dritte und vierte Absperrklemme 34, 48, 62 und 66 sind über Aktivierungsleitungen 90-100 und die beiden Längensensoren 84 und 86 über Signalleitungen 102 und 104 mit einer Steuer-/Regeleinheit 106 verbunden. Letztere Einheit 106 ist über eine Signalleitung 108 mit einer Eingabeeinheit 110 und über eine Aktivierungsleitung 112 mit einem Display 114 verbunden.

Die in Figur 1 dargestellte Vorrichtung wird folgendermaßen betrieben:
Es werden zunächst zwei wechselweise im anderen Schaltmodus befindliche Klemmenpaare, bestehend aus erster und vierter Absperrklemme 34 und 66 sowie zweiter und dritter Absperrklemme 48 und 62 gebildet, die jeweils im anderen Schaltrhythmus (offen/geschlossen) vorliegen. Wenn zunächst das erste Klemmenpaar 34, 66 nicht aktiviert, also geschlossen ist, befindet sich das zweite Klemmenpaar 48, 62 im aktivierten Zustand, ist also geöffnet. In dieser Stellung wird durch die Federkraft der Kompressionseinrichtung 70 die zweite Ausgleichskammer 60 in die zweite Bilanzkammerhälfte 42 entleert, wobei ein Rückfluß zur Substituatsquelle 52 durch die weiterlaufende Pumpe 54 verhindert wird. Da durch die vorgegebene Pumpenregelung genau die benötigte Flüssigkeitsmenge zur Füllung der Bilanzkammer 36 geliefert wird, wird letztere vollständig gefüllt, wobei die in der ersten Bilanzkammerhälfte 38 vorliegende Flüssigkeit vollständig durch die Filtratablaßleitung 44 in den Filtratbeutel 46 gefördert wird und die Füllung der Kammer durch eine beginnende Auffüllung der entsprechenden Ausgleichskammer kontrolliert werden kann. Aufgrund der Federkraft erfolgt die Entleerung der Ausgleichskammer relativ rasch (üblicherweise innerhalb einiger Sekunden),
während demgegenüber die Füllung der ersten Ausgleichskammer mit Filtrat im allgemeinen langsamer erfolgt, wobei letzter Füllvorgang von dem Verhältnis Blut-/Filtratfluß und dem tatsächlichen Blutfluß abhängt. Das Verhältnis der Förderraten während der beiden Füllvorgänge ist dabei abhängig vom mittleren Filtratfluß und dem gewählten Volumen, das sich vor Öffnen der Absperrklemme in der Ausgleichskammer befinden soll.

Im gleichen Takt füllt die Filtratpumpe 30 den Ausgleichskammerbeutel 32 gegen die Wirkung der Kompressionseinrichtung 80, da die entsprechende Einlaßklemme geschlossen ist und die Pumpe kontinuierlich fördert, wobei der Längensensor 84 die Ausdehnung des Beutels 32 bestimmt und überwacht. Das Signal wird über die Signalleitung 102 an die Steuereinrichtung 106 abgegeben, die von Taktbeginn an fortlaufend die Ausdehnung des Beutels überwacht und mit einem mit Hilfe der Eingabeeinrichtung 110 eingegebenen Soll-Wert vergleicht. Gegebenenfalls erfolgt eine Regelung der Pumpleistung der Filtratpumpe 30, wenn diese in Abweichung vom Soll-Wert zuviel oder zuwenig Filtrat fördert. Somit wird sichergestellt, daß jeweils ausreichend Filtrat am Ende des Takts in der Ausgleichskammer 32 zur Füllung der linken Bilanzkammerhälfte 38 zur Verfügung steht.

Um Bilanzierfehler auszuschalten, werden vorteilhafterweise sämtliche Klemmen 34, 48, 62 und 66 in den inaktiven Zustand bei dem Übergang von einem Takt zum nächsten geschaltet. Erst dann wird das jeweils andere Klemmenpaar aktiviert. In diesem Fall wird das erste Klemmenpaar 34/66 aktiviert, wodurch Filtrat in die linke Bilanzkammerhälfte 38 durch die Wirkung der Kompressionseinrichtung 68 gelangt und die frische Substituatlösung durch die Substituatablaßleitung 64 in den Blutkreislauf (Tropfkammer 26) verdrängt wird.

Während dieses Vorgangs werden die Pumpen 30 und 54 entsprechend dem vorigen Takt zueinander geschaltet, um das Filtrat in die Bilanzkammer zu fördern und um die zweite Ausgleichskammer 60 mit frischer Substituatlösung aus der Substituatquelle 52 zu füllen. Die Überwachung dieser zweiten Ausgleichskammer 60 durch den Längensensor 86 erfolgt in entsprechender Weise, wie vorstehend bei der ersten Ausgleichskammer 32 beschrieben ist.

Da in dieser Ausführungsform exakte Filtrat-/Substituatmengen gegeneinander bilanziert werden, also eine 1:1-Bilanzierung stattfindet, wird der Patient weder ultrafiltriert noch überwässert.

Um letzteres zu erreichen, wird folgendermaßen verfahren:
Um zu ultrafiltrieren, muß dem Patienten mehr Filtrat entnommen werden, als Substituatlösung zugeführt wird. Üblicherweise können dem Patienten bis zu 2 l/h Wasser und mehr entnommen werden, d.h. über die Behandlungszeit werden diese Mengen in entsprechenden Portionen entnommen.

Über die Eingabeeinheit 110 wird zunächst die zu ultrafiltrierende Menge und der Filtratfluß vorgegeben, wobei das Steuergerät 106 über die Aktivierungsleitungen 90 und 92 die Filtratpumpe 30 und die Substituatpumpe 54 entsprechend aktiviert und ihre jeweiligen Förderraten einstellt. Aus dieser vorgegebenen Ultrafiltrationsmenge errechnet das Steuergerät 106 die Takte, in denen Substituatflüssigkeit zur Substituatquelle und nicht zum Blutkreislauf 24 des Patienten gefördert wird. In diesem Takt wird - wie vorstehend beschrieben - beim Umschalten der Klemmen die Klemmenkonstellation auf der Substituatseite gegenüber dem vorhergehenden Takt beibehalten, während die Filtratklemme 34 geöffnet und die Abflußklemme 48 geschlossen werden. Zugleich wird die Förderrichtung der Substituatpumpe 54 umgekehrt, d.h., sie fördert auf die Substituatquelle 52 hin. Demzufolge erfolgt die Entleerung der Bilanzkammerhälfte 42 zur Ausgleichskammer 60 bzw. zur Substituatquelle 52, nicht jedoch über die Leitung 64 zum Blutkreislauf des Patienten. Damit eine eventuell noch in der Substituatausgleichskammer verbleibende kleine Restmenge nicht die vollständige Füllung der Bilanzkammer mit Filtrat verhindert, liegt die Federkraft auf der Substituatseite in diesem Fall unterhalb der Federkraft auf der Filtratseite (Federkennlinienschnitt).

Soll dagegen der Patient überwässert werden, wird der Überwässerungsgrad ebenfalls über die Eingabeeinrichtung 110 eingegeben, woraufhin die Taktzahl ermittelt wird, bei der Filtrat nicht zum Filtratbeutel 46, sondern vielmehr zur Filtratausgleichskammer 32 hin gefördert wird. Auch hier bleibt die Klemmenkonstellation beim Umschalten auf der Filtratseite gleich, während die Klemmen auf der Substituatseite umgeschaltet werden, d.h., die Klemme 62 wird geöffnet und die Klemme 66 wird geschlossen. Demzufolge wird also Substituatlösung in die rechte Bilanzkammerhälfte eingeführt, während Filtrat aus der linken Bilanzkammerhälfte in die Filtratausgleichskammer 32 verdrängt wird. Um das Filtrat vollständig in die Ausgleichskammer 32 zurückdrücken zu können, muß die Federkraft auf der Substituatseite trotz kleinerem Füllvolumen größer sein als auf der Gegenseite. Damit die Filtratpumpe und die Substituatpumpe 30 bzw. 54 nicht gegeneinander fördern, wird die Filtratpumpe 30 über die Leitung 90 inaktiviert und stillgesetzt. Im nächsten Takt erfolgt die Entleerung der Filtratausgleichskammer 32 zur Bilanzkammer 36 hin und die Verdrängung des Substituats aus der Bilanzkammer 36 ins Blut ohne Pumpen durch Federkraft, um den nächsten Zyklus wider normal beginnen zu können. Erst beim Umschalten in den nächsten Takt erfolgt also wieder die übliche Aktivierung der Klemmenpaare und der Pumpen.

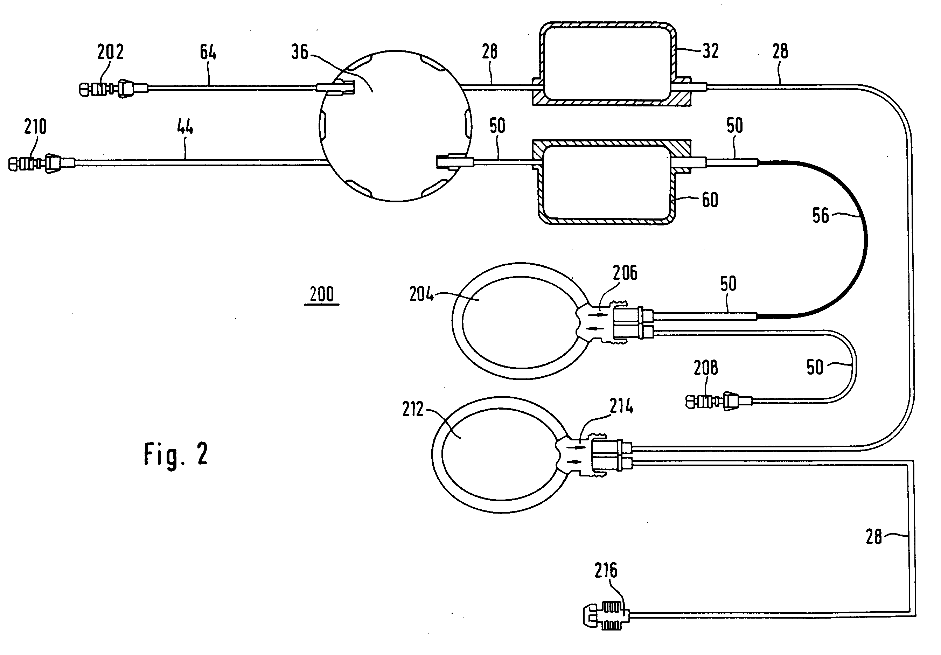

In Figur 2 ist ein Disposable 200 dargestellt, das im sterilen Zustand angeliefert und nach einmaliger Verwendung verworfen wird.

Nachstehend werden für die Teile, die denjenigen von Figur 1 entsprechen, die gleichen Bezugszeichen verwendet. Das Disposable 200 weist eine Bilanzkammer 36 auf, von der sich - von oben her gesehen - in Richtung Blutkreislauf die Substituatleitung 64 erstreckt. An deren Ende ist ein Konnektor 202 vorgesehen, der mit einem komplementären, nicht gezeigten Konnektorstück an der Tropfkammer 26 zusammenwirkt.

Weiterhin geht von der Oberseite die Substituatleitung 50 ab, in die das Heizteil 56 eingeschaltet ist, an das sich stromauf eine Pumpschleife 204 anschließt, die in die als Rollenpumpe ausgebildete Substituatpumpe 54 mit ihrem Befestigungsteil 206 eingelegt und dort fixiert werden kann. Das Ende der Substituatleitung 50 weist einen weiteren Konnektor 208 auf, der mit einem nicht gezeigten Konnektorstück verbunden werden kann, das an einem mit Substituatflüssigkeit gefüllten Beutel 52 angeordnet ist.

Weiterhin ist in die Substituatleitung 50 die Ausgleichskammer 60 eingeschaltet.

Von der Unterseite der Bilanzkammer 36 geht auf der einen Seite die Filtratablaßleitung 44 ab, deren Ende ebenfalls mit einem Konnektor 210 versehen ist, der mit einem nicht gezeigten Konnektorstück zusammenwirkt, das am Filtratbeutel 46 angebracht ist.

Auf der anderen Seite der Bilanzkammer 36 geht die Filtratzuleitung 28 ab, in die die Filtratausgleichskammer 32 eingeschaltet ist. Stromauf der Ausgleichskammer 32 ist eine weitere Pumpschleife 212 mit einem entsprechenden Befestigungsteil 214 vorgesehen, die in die ebenfalls als Rollenpumpe ausgebildete Filtratpumpe 30 eingelegt und dort fixiert werden kann.

Das Ende der Filtratleitung 28 ist mit einem Konnektor 216 versehen, der mit einem nicht gezeigten Konnektorstück verbunden werden kann, das beispielsweise am Filter 12 angeordnet ist.

Wie bereits eingangs beschrieben, ist die erfindungsgemäße Bilanzierungseinrichtung nicht auf die Anwendung bei der Plasma- oder Hämofiltration beschränkt. Vielmehr kann sie auch auf andere Blutbehandlungsmethoden oder auf die kontinuierliche ambulante Peritonealdialyse (CAPD) übertragen werden.

Im übrigen ist die Bilanzkammer selbst Gegenstand einer eigenen parallelen Patentanmeldung, die am gleichen Tage (unser Zeichen FR1869) hinterlegt worden ist. Insofern wird auf deren Offenbarung Bezug genommen.

## Patentansprüche

1. Vorrichtung zur Behandlung von Blut in einem extrakorporalen Blutkreislauf, der durch die Blutkammer (16) eines Filters (12) gelegt ist, das durch eine semipermeable Membran (14) in die Blutkammer (16) und eine Filtratkammer (18) geteilt ist, mit einer Filtratleitung (28), die von der Filtratkammer (18) abgeht und in die eine Filtratpumpe (30) eingeschaltet ist, mit einer Substituatquelle (52), von der eine Substituatleitung (50) abgeht, in die eine Substituatpumpe (54) eingeschaltet ist, mit einer Bilanzierkammer (36), die durch eine flexible Wand (40) in eine erste und eine zweite Bilanzierkammerhälfte (38 bzw. 42) geteilt ist, wobei die erste Hälfte (38) mit der Filtratleitung (28) und die zweite Hälfte (42) mit der Substituatleitung (50) verbunden sind, mit einer Filtratauslaßleitung (44), die von der ersten Bilanzkammerhälfte (38) zu einem Auslaß abgeht, und einer Substituatauslaßleitung (64), die von der zweiten Bilanzkammerhälfte (42) abgeht und mit dem extrakorporalen Blutkreislauf zu verbinden ist, einer ersten Absperrklemme (34), die an der Filtratleitung (28) angeordnet ist, einer zweiten Filtratklemme (48), die an der Filtratauslaßleitung (44) angeordnet ist, einer dritten Absperrklemme (62), die an der Substituatleitung (50) angeordnet ist, und einer vierten Absperrklemme (66), die an der Substituatauslaßleitung (64) angeordnet ist, und mit einer Steuereinheit (106), die die pumpen (22, 54) und die Absperrklemmen (34, 48, 62, 66) in vorbestimmter Weise aktiviert, dadurch gekennzeichnet, daß in der Filtratleitung (28) und der Substituatleitung (50) jeweils eine Ausgleichskammer (32 bzw. 60) stromauf der ersten bzw. dritten Absperrklemme (34 bzw. 62) vorgesehen ist, deren Aufnahmevolumen jeweils größer ist als das Innenvolumen der Bilanzkammer (36), und daß die Ausgleichskammern (32, 60) jeweils von stetig Druck auf sie ausübenden Kompressionseinrichtungen (68 bzw. 70) beaufschlagt sind.

2. Vorrichtung nach Anspruch 1, dadurch gekennzeichnet, daß die Kompressionseinrichtung (68, 70) aus zwei Platten (72, 74; 76, 78) besteht, wobei die beiden platten (72 und 74 bzw. 76 und 78) durch eine Feder (80, 82) zusammengespannt sind.

3. Vorrichtung nach Anspruch 1 oder 2, dadurch gekennzeichnet, daß an der Kompressionseinrichtung (68, 70) ein Längensensor (84, 86) vorgesehen ist, mit dem die Erweiterung der Ausgleichskammer (32, 60) bestimmbar ist.

4. Vorrichtung nach Anspruch 1, dadurch gekennzeichnet, daß die Steuereinheit (106) wechselweise entgegengesetzt die Absperrklemmen (34, 66) und (48, 42) in einen der beiden Takte schaltet und in der Füllphase die pumpen (30, 54) jeweils in eine erhöhte vorbestimmte Förderrate umschaltet.

5. Vorrichtung nach einem der Ansprüche 1 bis 4, dadurch gekennzeichnet, daß zur Ultrafiltration bei mit Substituat gefüllter Bilanzkammerhälfte (42) die Klemme (62) geöffnet und die Klemme (66) geschlossen bleiben, wenn die Klemmen (34 und 48) umgeschaltet worden sind, d.h. in die offene bzw. geschlossene Phase überführt worden sind, und daß die Drehrichtung der Substituatpumpe (54) in diesem Takt umgekehrt ist.

6. Vorrichtung nach einem der Ansprüche 1 bis 4, dadurch gekennzeichnet, daß zur Überwässerung bei mit Filtrat gefüllter Bilanzkammerhälfte (38) die Klemme (34) geöffnet und die Klemme (48) geschlossen bleiben, wenn die Klemmen (62 und 66) umgeschaltet worden sind, d.h. in die offene bzw. geschlossene Phase überführt worden sind, und daß die Filtratpumpe (30) in diesem Takt stillgesetzt ist.

7. Vorrichtung nach Anspruch 6, dadurch gekennzeichnet, daß die Konstante der Feder (80) der Kompressionseinrichtung (68) größer ist als die Konstante der Feder (82) der Kompressionseinrichtung (70).

8. Vorrichtung nach einem der Ansprüche 1 bis 7, dadurch gekennzeichnet, daß der Längensensor (84, 86) im Fülltakt die Veränderung des Abstandes der plattenpaare (72, 74; 76, 78) voneinander zeitabhängig ermittelt und ein der Längenveränderung proportionales Signal an die Steuereinheit (106) übermittelt, die diesen Ist-Wert mit einem Soll-Wert vergleicht und bei einer vorbestimmten Abweichung die Pumprate der jeweils im Füllbetrieb befindlichen Pumpe (30, 54) entsprechend anpaßt.

9. Disposable (200) zur Verwendung in einer bilanzierenden Vorrichtung, insbesondere in der Plasmafiltrationsvorrichtung gemäß Anspruch 1, gekennzeichnet durch eine Bilanzkammer (36), die durch eine flexible Wand (40) in zwei Bilanzkammerhälften (38), (42) geteilt ist, eine Filtratleitung (28), die sich zur ersten Bilanzkammerhälfte (38) erstreckt und in die eine Pumpschleife (212) zum Einlegen in eine als Rollenpumpe ausgebildete Filtratpumpe (30) sowie stromab hierzu eine erste Ausgleichskammer (32) eingeschaltet sind, eine von der ersten Bilanzkammerhälfte (38) abgehende Filtratauslaßleitung (44), eine Substituatleitung (50), die sich zur zweiten Bilanzkammerhälfte (42) erstreckt und in die eine zweite Pumpschleife (204) zum Einlegen in eine als Rollenpumpe ausgebildete Substituatpumpe (54) sowie ein Heizungsrohr (56) und stromab hierzu eine zweite Ausgleichskammer (60) eingeschaltet sind, eine von der zweiten Bilanzkammerhälfte (42) abgehende Substituatauslaßleitung (64) und durch Konnektoren (202, 208, 210, 216), die jeweils mit den Enden sämtlicher Leitungen (28, 44, 50, 64) verbunden sind.

## Claims

1. Apparatus for treating blood in an extracorporeal blood circuit being in communication with the blood chamber (16) of a filter (12) which by means of a semipermeable membrane (14) is divided into the blood chamber (16) and a filtrate chamber (18), said apparatus comprising a filtrate conduit (28) branching off the filtrate chamber (18) and into which a filtrate pump (30) is switched, comprising a substituate source (52) from which a substituate conduit (50) branches off into which a substituate pump (54) is switched, comprising a balancing unit (36) which by means of a flexible wall (40) is divided into a first and a second balancing unit half (38, 42), with the first half (38) being connected with the filtrate conduit (28) and the second half (42) with the substituate conduit (50), comprising a filtrate outlet conduit (44) branching off the first balancing unit half (38) to an outlet, and comprising a substituate outlet conduit (64) branching off the second balancing unit half (42) and being to be connected with the extracorporeal blood circuit, comprising a first shut-off clamp (34) being mounted to the filtrate conduit (28), a second filtrate clamp (48) being mounted to the filtrate outlet conduit (44), a third shut-off clamp (62) which is mounted to the substituate conduit (50), and a fourth shut-off clamp (66) being mounted to the substituate outlet conduit (64), and further comprising a control unit (106) which activates the pumps (22, 54) and the shut-off clamps (34, 48, 62, 66) in predetermined manner, characterized in that one equalizing chamber each (32, resp. 60) is provided in the filtrate conduit (28) and in the substituate conduit (50) upstream of the first, resp. third shut-off clamp (34, resp. 62), the take-up volume of the equalizing chambers each being larger than the inner volume of the balancing unit (36), and that the equalizing chambers (32, 60) are respectively acted upon by compression units (68, resp. 70) exerting constant pressure thereon.

2. Apparatus according to claim 1, charcterized in that the compression device (68, 70) consists of two plates (72, 74; 76, 78), with the two plates (72 and 74, resp. 76 and 78) being tensed together by means of a spring (80, 82).

3. Apparatus according to claim 1 or 2, characterized in that at the compression device (68, 70) a length sensor (84, 86) is provided by means of which the expansion of the balancing unit (32, 60) is determinable.

4. Apparatus according to claim 1, characterized in that the control unit (106) switches the closing clamps (34, 66) and (48, 42) alternately opposite into one of the two phases and in the filling phase switches the pumps (30, 54) respectively over to an increased predetermined filling rate.

5. Apparatus according to one of claims 1 to 4, characterized in that for ultrafiltration in case of a balancing unit half (42) filled with substituate the clamp (62) remains open, and the clamp (66) remains closed when the clamps (34 and 48) have been switched over, i.e. have been switched into the open, resp. closed phase, and that the direction of rotation of the substituate pump (54) in this phase is reversed.

6. Apparatus according to any of claims 1 to 4, characterized in that for over-soaking in case of a balancing unit half (38) filled with filtrate the clamp (34) remains open, and the clamp (48) remains closed when the clamps (62 and 66) have been switched over, i.e. have been switched into the open, resp. closed phase, and that the filtrate pump (30) in this phase is shut down.

7. Apparatus according to claim 6, characterized in that the constant of the spring (80) of the compression device (68) is larger than the constant of the spring (82) of the compression device (70).

8. Apparatus according to any of claims 1 to 7, characterized in that the length sensor (84, 86) in the filling phase determines time dependently the change of the interspace of the pairs of plates (72, 74; 76, 78) and submits a signal proportional to the change in length to the control unit (106) which compares the actual value with a set value and adapts accordingly at a predetermined deviation the pump rate of the pump (30, 54) being respectively in filling operation.

9. Disposable (200) for use in a balancing apparatus, in particular in the plasma filtration apparatus according to claim 1, characterized by a balancing unit (36) being divided by means of a flexible wall (40) into two balancing unit halves (38), (42), one filtrate conduit (28) which extends to the first balancing unit half (38) and into which a pump loop (212) for inserting into a filtrate pump (30) embodied as roller pump as well as downstream hereof a first equalizing chamber (32) are switched, a filtrate outlet conduit (44) branching off the first balancing unit half (38), a substituate conduit (50) extending to the second balancing unit half (42) and into which a pump loop (204) for inserting into a substituate pump (54) embodied as roller pump as well as a heating tube (56) and downstream hereof a second equalizing chamber (60) are switched, a substituate outlet conduit (64) branching off the second balancing unit half (42), and by connectors (202, 208, 210, 216), which are respectively connected to the ends of all conduits (28, 44, 50, 64).

## Revendications

1. Dispositif pour le traitement du sang dans un circuit extracorporel de circulation du sang, qui passe par la chambre à sang (16) d'un filtre (12), qui est divisée par une membrane semi-perméable (14) en la chambre à sang (16) et une chambre à filtrat (18), et comportant une canalisation (28) pour le filtrat, qui part de la chambre à filtrat (18) et dans laquelle est montée une pompe à filtrat (30), et comportant une source (52) délivrant un produit de substitution, à partir de laquelle s'étend une canalisation (50) pour le produit de substitution, dans laquelle est montée une pompe (54) pour le produit de substitution, et comportant une chambre d'équilibrage (36), qui est divisée par une paroi flexible (40) en des première et seconde moitiés (38 et 42) de la chambre d'équilibrage, dont la première moitié (38) est reliée à la canalisation (28) pour le filtrat et dont la seconde moitié (42) est reliée à la canalisation (50) pour le produit de substitution, et comportant une canalisation (44) de sortie du filtrat, qui part de la première moitié (38) de la chambre d'équilibrage en direction d'une sortie, et une canalisation (64) de sortie du produit de substitution, qui part de la seconde moitié (42) de la chambre d'équilibrage et doit être reliée au circuit extracorporel de circulation du sang, un premier étranglement de sectionnement (34), qui est disposé dans la canalisation (28) pour le substrat, un second étranglement (48) pour le filtrat, qui est disposé dans la canalisation (44) de sortie pour le filtrat, un troisième étranglement de sectionnement (62), qui est disposé dans la canalisation (50) pour le produit de substitution et un quatrième étranglement de sectionnement (66), qui est disposé dans la canalisation (64) de sortie pour le produit de substitution, et comportant une unité de commande (106), qui active d'une manière prédéterminée les pompes (22,54) et les étranglements de sectionnement (34,48,62,66), caractérisé en ce que dans les canalisations (28) pour le filtrat et dans la canalisation (50) pour le produit de substitution sont prévues des chambres respectives de compensation (32 et 60) disposées en amont des premier et troisième étranglements de sectionnement (34 et 62), dont les volumes de réception sont respectivement supérieurs au volume intérieur de la chambre d'équilibrage (36), et que les chambres de compensation (32,60) sont chargées respectivement par des dispositifs de compression (68 et 70), qui appliquent en permanence une pression à ces chambres.

2. Dispositif selon la revendication 1, caractérisé en ce que le dispositif de compression (68,70) est constitué de deux plaques (72, 74; 76, 78), les deux plaques (72 et 74 ou 76 et 78) étant serrées l'une contre l'autre par un ressort (80, 82).

3. Dispositif selon la revendication 1 ou 2, caractérisé en ce que dans le dispositif de compression (68, 70) est prévu un capteur de longueur (84,86), au moyen duquel peut être déterminée l'extension de la chambre de compensation (32,60).

4. Dispositif selon la revendication 1, caractérisé en ce que l'unité de commande (106) est commutée alternativement en des sens opposés, les étranglements de sectionnement (34,66) et (48,42) suivant l'un des deux cycles et, pendant la phase de remplissage, commute les pompes (30,54) respectivement sur un débit de refoulement prédéterminé accru.

5. Dispositif selon l'une des revendications 1 à 4, caractérisé en ce que pour l'ultrafiltration, dans le cas où la moitié (42) de la chambre d'équilibrage est remplie par le produit de substitution, l'étranglement (62) est ouvert et l'étranglement (66) reste fermé, lorsque les étranglements (34 et 48) ont été commutés, c'est-à-dire ont été amenés dans la position ouverte ou fermée, et que le sens de rotation de la pompe (54) pour le produit de substitution est inversé pendant ce cycle.

6. Dispositif selon l'une des revendications 1 à 4, caractérisé en ce que pour la surhydratation dans le cas où la moitié (38) de la chambre d'équilibrage, remplie par le filtrat, l'étranglement (34) est ouvert et l'étranglement (48) reste fermé, lorsque les étranglements (62 et 66) ont été commutés, c'est-à-dire ont été amenés dans la position ouverte ou fermée, et que la pompe (30) pour le filtrat est arrêtée au cours de ce cycle.

7. Dispositif selon la revendication 6, caractérisé en ce que la constante du ressort (80) du dispositif de compression (68) est supérieure à la constante du ressort (82) du dispositif de compression (70).

8. Dispositif selon l'une des revendications 1 à 7, caractérisé en ce que le capteur de longueur (84,86) détermine, lors du cycle de remplissage, la variation de la distance entre les plaques des couples de plaques (72,74; 76,78) indépendamment du temps, et transmet un signal proportionnel à la variation de longueur à l'unité de commande (106), qui compare cette valeur réelle à une valeur de consigne et, dans le cas d'un écart prédéterminé, le débit de la pompe (30,54) qui effectue respectivement l'opération de remplissage, est adapté de façon correspondante.

9. Article jetable (200) destiné à être utilisé dans un dispositif d'équilibrage, notamment dans le dispositif de filtration de plasma selon la revendication 1, caractérisé par une chambre d'équilibrage (36), qui est divisée par une paroi flexible (40) en deux moitiés (38,42), une canalisation (28) pour le filtrat, qui s'étend jusqu'à la première moitié (38) de la chambre d'équilibrage et dans laquelle sont branchées une boucle de pompe (212) destinée à être insérée dans une pompe (30) pour le filtrat, réalisé sous la forme d'une pompe à rouleaux, ainsi que, en aval de cette pompe, une première chambre de compensation (32), une canalisation (44) de sortie du filtrat, qui s'étend à partir de la première moitié (38) de la chambre d'équilibrage, une canalisation (50) pour le produit de substitution, qui s'étend jusqu'à la seconde moitié (42) de la chambre d'équilibrage et dans laquelle sont branchées une seconde boucle de pompe (204) destinée à être insérée dans une pompe (54) pour le produit de substitution, réalisée sous la forme d'une pompe à rouleaux, ainsi qu'un tube de chauffage (56) et, en aval de cela, une seconde chambre de compensation (60), une canalisation (64) de sortie du premier substituant, qui s'étend à partir de la seconde moitié (42) de la chambre d'équilibrage, et des raccords (202,208,210,216), qui sont reliés respectivement aux extrémités de toutes les canalisations (28,44,50,64).
